# EUROPÄISCHE PATENTANMELDUNG

(11) **EP 2 145 643 A1**
(43) Veröffentlichungstag der Anmeldung: **20.01.2010**
(21) Anmeldenummer: 08013064.4
(22) Anmeldetag: 19.07.2008
(51) Int. Cl.: A61M 11/04, A61M 15/00, B65D 75/30

(54) **Portioniertes Arzneimittel**

(71) Anmelder: Storz, Markus, 78532 Tuttlingen (DE)
(72) Erfinder: Storz, Markus, 78532 Tuttlingen (DE)
(74) Vertreter: Späth, Dieter

(57) **Zusammenfassung**

Das freihändige Einfüllen von verdampfbaren Substanzen in die Füllkammer eines Inhalators durch den Anwender wird insbesondere den Anforderungen einer medizinischen Anwendung nur bedingt gerecht. Grundsätzlich besteht die Gefahr der Fehldosierung. Außerdem sind viele Patienten aufgrund ihrer Erkrankung in ihrer Feinmotorik beeinträchtigt, so daß sie gar nicht in der Lage sind, derart diffizile Aufgaben auszuführen.

Für den medizinischen Einsatz wird daher ein möglichst billiges, vom Apotheker oder der Pharmaindustrie bereits mit der gewünschten Substanz vordosiertes und standardisiertes Teil für den Einmalgebrauch benötigt.

Der Erfindung schlägt dafür ein Portionspad vor, welches vom Hersteller bereits vordosiert wurde und daher eine einfache und sichere Anwendung gewährleistet.

Diese Aufgabe wird erfindungsgemäß durch die im Patentanspruch 1 aufgeführten Merkmale (Portion eines durch Hitzeeinwirkung verdampfbaren, durch Inhalation wirksamen Arzneimittels, dosiert für eine Anwendung und handhabbar hergerichtet als ein Teil zur Verwendung in einem Inhalator) gelöst.

## Beschreibung

Bei der hier vorliegenden Erfindung handelt es sich um eine sinnvolle Ergänzung des vom selben Autor gehaltenen Patents (DE 198 03 376 bzw. EP 0933093 und US 6,513,524). Der dort beschriebene Inhalator dient zur thermischen Verdampfung und anschliessenden Inhalation von verdampfbaren, also unter Hitzeeinwirkung aerosolbildenden Substanzen. Diese Substanzen werden nach dem heutigen Stand der Technik entweder auf ihrem natürlichen Träger, z.B. den nicht verdampfbaren Pflanzenfasern eines Krauts bzw. anderer Pflanzenteile, oder bei flüssigen, bzw. pulverisierten Substanzen auf einem künstlichen Träger wie z.B. einem Pad aus gepresstem Edelstahldrahtgestrick in eine Füllkammer verbracht. Dieses Edelstahldrahtgestrick ist zur Wiederverwendung vorgesehen, die Portion muß vom Anwender aufgebracht und das Pad nach Benutzung für die nächste Anwendung gereinigt werden. Die Füllkammer, gefüllt mit Pflanzenmaterial bzw. dem Edelstahldrahtgestrick und den darauf aufgebrachten Substanzen wird anschließend mit Heißluft durchströmt, wodurch die Substanzen verdampfen bzw. ausdünsten und in die Heißluft übergehen. Diese, z.B. mit Aromen und/oder Wirkstoffen versetzte Luft wird, nachdem Sie auf eine angenehme Temperatur heruntergekühlt ist, inhaliert, wobei die Wirkstoffe über die Lungenbläschen in den Blutkreislauf gelangen.

Das freihändige Einfüllen der Substanzen in die Füllkammer durch den Anwender wird insbesondere den Anforderungen einer medizinischen Anwendung nur bedingt gerecht. Grundsätzlich besteht die Gefahr der Fehldosierung. Substanzen, die im Milligrammbereich verabreicht werden sollen, können zuhause vom Patienten kaum zuverlässig abgewogen werden. Werden die Substanzen zur besseren Dosierbarkeit und/oder zur gleichmäßigeren Verteilung auf dem Edelstahldrahtgestrick mit Füllstoffen oder Lösungsmitteln versehen, so ist es z.B. im Fall von Alkohol als Lösungsmittel erforderlich, diesen vorab bei niedrigen Temparaturen zu verdampfen, also eine Separation herbeizuführen, um anschließend die gewünschte Substanz zur Verdampfung und Inhalation zu bringen.
Viele Patienten sind aufgrund ihrer Erkrankung in ihrer Feinmotorik oder anderweitig beeinträchtigt, so daß sie gar nicht in der Lage sind, derart diffizile Aufgaben auszuführen.

Der Erfindung liegt die Aufgabe zugrunde, eine Möglichkeit zur einfachen Befüllung vorzuschlagen, welche Fehldosierungen zuverlässig verhindert und eine einfache und sichere Anwendung gewährleistet.

Diese Aufgabe wird erfindungsgemäß durch die im Patentanspruch 1 aufgeführten Merkmale (Portion eines durch Hitzeeinwirkung verdampfbaren, durch Inhalation wirksamen Arzneimittels, dosiert für eine Anwendung und handhabbar hergerichtet als ein Teil zur Verwendung in einem Inhalator) gelöst.
Die Portion, bzw. ihre Herrichtung als ein Teil oder ein Stück kann auch als Pad oder Portionspad bezeichnet werden.
Dieses Teil könnte zum Beispiel ein Beutel aus zwei Lagen sein, in welchem sich das Arzneimittel befindet. Weiter könnte es sich z.B. um ein getränktes oder das Arzneimittel in sonstiger Weise enthaltendes Substrat handeln. Das Arzneimittel könnte auch als Pulver in einem Gewirk oder Schwamm enthalten sein. In jedem Fall kann man das Teil, welches das Arzneimittel enthält in die Hand nehmen, egal wie es aufgebaut ist. Der Einfachkeit halber wird dieses Teil in der Beschreibung als "Portionspad" bezeichnet.

Gegenstand der Erfindung ist also ein preisgünstiges, vom Apotheker oder der Pharmaindustrie bereits mit der gewünschten Substanz (Arzneimitte)I vordosiertes Teil im Sinne eines einstückigen, in die Hand nehmbaren Gegenstands für den Einmalgebrauch in einem Inhalator.

Die mit der Erfindung erzielten Vorteile bestehen insbesondere darin, dass das Befüllen der Füllkammer für den Anwender wesentlich vereinfacht wird. Es braucht nur noch das Portionspad eingelegt werden. Außerdem ist die Gefahr der Fehldosierung durch den Anwender praktisch ausgeschlossen, da dies durch Fachleute bereits vorher erledigt wird und das Portionspad zur Einmalverwendung vorgesehen werden kann. Eine Separation von Lösungsmitteln bei flüssigen Substanzen durch den Anwender ist dann ebenfalls überflüssig.
Eine vorteilhafte Ausgestaltung der Erfindung ist im Patentanspruch 3 angegeben. Dieses Pad könnte z.B. aus einer Lage eines saugfähigen, luft- und dampfdurchlässigen Trägermaterials in Scheibenform bestehen, auf welches die Substanz durch den Hersteller dosiert aufgebracht wurde. Die oben beschriebene Separation wäre unnötig, wenn flüssige Substanzen vom Hersteller mittels eines geigneten Apparates dosiert und lösungsmittelfrei auf ein Pad aufgesprüht würden. Bei der Verwendung von lösungsmittelhaltigen Substanzen auf dem Pad könnten die Hersteller die Separation des Lösungsmittels bereits vor der Auslieferung an den Anwender besorgen.
Eine weitere vorteilhafte Ausgestaltung der Erfindung ist im Patentanspruch 4 angegeben. Feste Substanzen wie z.B. zerkleinerte Pflanzenteile oder Pulver werden zwischen zwei Lagen eines luft- und dampfdurchlässigen Stoffes, die am umlaufenden Rand miteinander verbunden sind, platziert.
Eine weitere vorteilhafte Ausgestaltung der Erfindung ist im Patentanspruch 5 angegeben. Durch die Verwendung eines Substanzträgers zwischen den Lagen können z. B. auch klebrige/harzige Substanzen im Portionspad verdampft und z.B. aufeinandergestapelt gelagert werden ohne aneinander zu kleben.
Eine weitere vorteilhafte Ausgestaltung der Erfindung ist im Patentanspruch 6 angegeben. Durch Verpacken unter Schutzatmosphäre können auch Luft- bzw. Sauerstoffempfindliche Substanzen für das Portionspad Verwendung finden. Lichtempfindliche Substanzen können durch lichtundurchlässige Verpackungen geschützt werden.

Die Erfindung wird nachfolgend anhand eines in der Zeichnung dargestellten Ausführungsbeispiels näher erläutert. Es zeigen:
- Figur 1: einen Achsschnitt durch den erfindungsgemäßen Portionspad mit einer Kräuter- oder Pulverfüllung;
- Figur 2: einen Achsschnitt durch den erfindungsgemäßen Portionspad mit einem zwischen den Lagen positioniertem saugfähigen Stoff zur Aufnahme von flüssigen Substanzen;
- Figur 3: einen Achsschnitt durch den erfindungsgemäßen Portionspad mit einem zwischen den Lagen positioniertem saugfähigen Stoff zur Aufnahme von flüssigen Substanzen, sowie einer Schutzverpackung aus luftundurchlässiger Folie, notwendig zum Verpacken unter Schutzatmosphäre;
- Figur 4: einen Achsschnitt durch den erfindungsgemäßen Portionspad, mit einem zwischen den Lagen positioniertem saugfähigen Stoff zur Aufnahme von flüssigen Substanzen, positioniert in einer für die Aufnahme von Pads vorgesehenen Füllkammer;
- Figur 5: eine perspektivische Ansicht eines Kräuterportionspads;

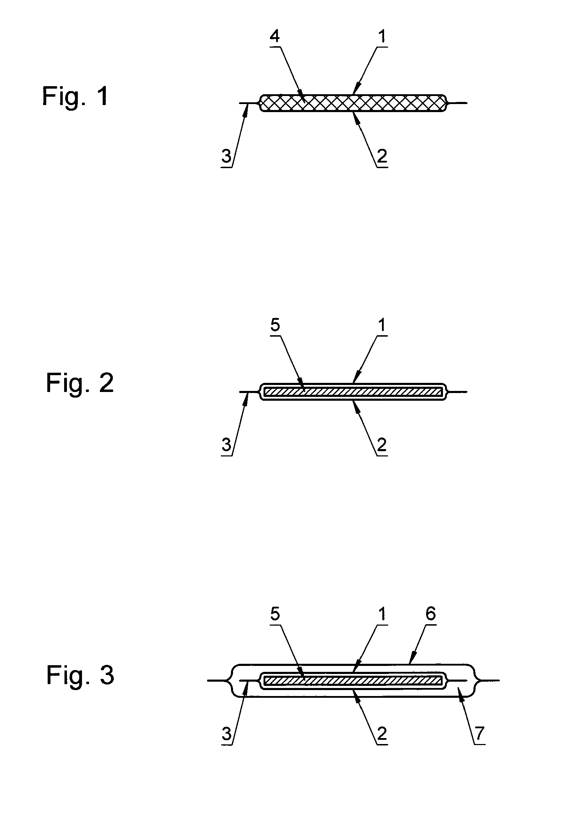

Auf die zeichnerische Darstellung eines einlagigen, scheibenförmigen Pads mit aufgebrachter Substanz wurde verzichtet, da leicht vorstellbar. Das in Figur 1 dargestellte, erfindungsgemäße Portionspad nach Patentanspruch 2 weist eine obere Lage (1) und eine untere Lage (2) eines luft- und dampfdurchlässigen Stoffes auf, wie z.B. Teebeutelpapier, welche in der Randzone (3) miteinander verbunden sind. Dazwischen befindet sich die Füllung (4) aus zerkleinerten Kräutern oder sonstigen zerkleinerten Feststoffen bzw. Pulvern.
Das in Figur 2 dargestellte, erfindungsgemäße Portionspad nach Patentanspruch 3 weist eine obere Lage (1) und eine untere Lage (2) eines luft- und dampfdurchlässigen Stoffes auf wie z.B. Teebeutelpapier, welche in der Randzone (3) miteinander verbunden sind. Dazwischen befindet sich ein saugfähiger und luftdurchlässiger Stoff (5) wie z.B. Papiervlies zur Aufnahme von flüssigen Substanzen bzw. Lösungen.
In Figur 3 wird ein Portionspad wie in Figur 2 dargestellt, welches unter Schutzgas (7) nach Patentanspruch 4 in eine zusätzliche, luftundurchlässige Folie (6) wie z.B. Aluminium-Kunststoffverbundfolie verpackt wurde.

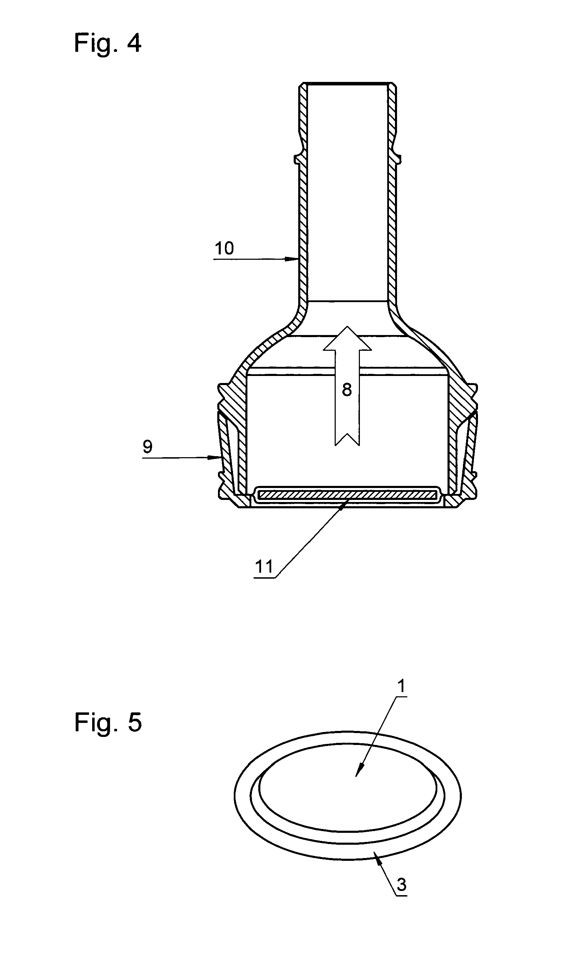

Figur 4 zeigt ein erfindungsgemäßes Portionspad positioniert in der dafür vorgesehenen Füllkammer (9) eines im Übrigen nicht dargestellten Inhalators, wobei der aufgeschraubte Füllkammerdeckel (10) das Portionspad an der dafür vorgesehenen Stelle im korrekten Abstand zur Hitzequelle fixiert.
Figur 5 zeigt ein erfindungsgemäßes Portionspad nach Figur 2 in perspektivischer Ansicht.

### Bezugszeichenliste:

- 1: obere Lage
- 2: untere Lage
- 3: Randzone
- 4: Kraut oder Pulver
- 5: saugfähiger Stoff
- 6: Folienhülle
- 7: Schutzgaszone
- 8: Durchströmungsrichtung
- 9: Füllkammer
- 10: Füllkammerdeckel
- 11: Portionspad

## Patentansprüche

1. Portion eines durch Hitzeeinwirkung verdampfbaren, durch Inhalation wirksamen Arzneimittels, dosiert für eine Anwendung und handhabbar hergerichtet als ein Teil zur Verwendung in einem Inhalator.

2. Portion nach Patentanspruch 1,
**dadurch gekennzeichnet,**
**dass** das Arzneimittel ein Cannabinoid oder ein Opioid aufweist.

3. Portion nach Patentanspruch 1,
**dadurch gekennzeichnet,**
**dass** sich die verdampfbaren Substanzen auf einer Lage eines luft- und dampfdurchlässigen Stoffes befinden.

4. Portion nach Patentanspruch 1,
**dadurch gekennzeichnet,**
**dass** sich die verdampfbaren Substanzen zwischen zwei schützenden Lagen eines luft- und dampfdurchlässigen Stoffes befinden, die am umlaufenden Rand miteinander verbunden sind.

5. Portion nach Patentanspruch 1,
**dadurch gekennzeichnet,**
**dass** sich zwischen zwei schützenden Lagen eines luft- und dampfdurchlässigen Stoffes eine weitere Lage befindet, welcher zur Aufnahme von flüssigen Substanzen bzw. Lösungen dient.

6. Portion nach Patentanspruch 1,
**dadurch gekennzeichnet,**
**dass** die Portion unter Schutzatmosphäre verpackt ist.

7. Verwendung einer Portion nach einem der vorhergehenden Ansprüche in einem Inhalator.

## Geänderte Patentansprüche

### Geänderte Patentansprüche gemäss Regel 137(2) EPÜ.

**1.**
Inhalator mit einer heißluftdurchströmbaren Füllkammer (9), **dadurch gekennzeichnet, dass** in die Füllkammer (9) ein Portionspad (11) mit einer Portion eines durch Hitzeeinwirkung verdampfbaren, durch Inhalation wirksamen Arzneimittels, dosiert für eine Anwendung und handhabbar hergerichtet als ein Teil, eingelegt ist.

**2.**
Inhalator nach Patentanspruch 1, **dadurch gekennzeichnet, dass** das Arneimittel ein Cannabinoid oder ein Opioid aufweist.

**3.**
Inhalator nach Patentanspruch 1, **dadurch gekennzeichnet, dass** sich die verdampfbaren Substanzen auf einer Lage eines luft- und dampfdurchlässigen Stoffes befinden.

**4.**
Inhalator nach Patentanspruch 1, **dadurch gekennzeichnet, dass** sich die verdampfbaren Substanzen zwischen zwei schützenden Lagen eines luft- und dampfdurchlässigen Stoffes befinden, die am umlaufenden Rand miteinander verbunden sind.

**5.**
Inhalator nach Anspruch 5, **dadurch gekennzeichnet, dass** das Portionspad (11) am umlaufenden Rand (3) mit einem Füllkammerdeckel (10) in der Füllkammer (9) des Inhalators fixiert ist.

**6.**
Inhalator nach Patentanspruch 1, **dadurch gekennzeichnet, dass** sich zwischen zwei schützenden Lagen eines luft- und dampfdurchlässigen Stoffes eine weitere Lage befindet, welcher zur Aufnahme von flüssigen Substanzen bzw. Lösungen dient.

**7.**
Inhalator nach Patentanspruch 1 oder 2, **dadurch gekennzeichnet, dass** die Portion unter Schutzatmosphäre verpackt ist.

**8.**
Verfahren zur thermischen Verdampfung eines Arzneimittels, **dadurch gekennzeichnet, dass** ein Portionspad (11) mit einer Portion eines durch Hitzeeinwirkung verdampfbaren, durch Inhalation wirksamen Arzneimittels, dosiert für eine Anwendung und handhabbar hergerichtet als ein Teil von Heißluft durchströmt wird, wodurch das Arzneimittel verdampft und die das Portionspad (11) durchströmende Luft mit Aromen und/oder Wirkstoffen versetzt wird.
